# EUROPEAN PATENT APPLICATION

(11) **EP 1 428 518 A2**
(43) Date of publication of application: **16.06.2004**
(21) Application number: 03027318.9
(22) Date of filing: 26.11.2003
(51) Int. Cl.: A61K 7/16

(54) **Method for optimizing fluoride uptake of teeth by applying a liquid dentifrice composition**

(30) Priority: 12.12.2002 US 317505
(71) Applicant: Colgate-Palmolive Company, New York, N.Y. 10022-7499 (US)
(72) Inventor: Joziak, Marilou T., South River, NJ 08882 (US); Fisher, Steven W., Middlesex, NJ 08846 (US)
(74) Representative: UEXKÜLL & STOLBERG

(57) **Abstract**

A method is provided for optimizing fluoride ion uptake on teeth wherein a liquid dentifrice composition which contains in an orally acceptable vehicle an abrasive and a fluoride ion releasable fluoride at a concentration sufficient to release 3000 to 6000 ppm fluoride ion, the viscosity of the composition ranging from 10,000 to 50,000 cps. Fluoride ion uptake and its accompanying anticaries benefit is optimized when the composition is administered during tooth brushing.

## Description

### Field of the Invention

This invention is directed to a liquid dentifrice composition containing fluorine ion releasable compounds effective as anticaries agents and more particularly to a liquid dentifrice composition for fluoridating teeth providing optimized tooth uptake of fluoride ions during brushing.

### The Prior Art

Liquid dentifrice compositions are formulated at concentration levels of water and humectant which are substantially higher than those used to prepare dentifrice pastes or gels. The liquid dentifrices are distinguished from mouth rinses as they contain abrasive materials which function to remove pellicle film deposits from tooth surfaces. The pellicle film deposits are tightly adherent and contain brown or yellow pigments which impart an unsightly appearance to the teeth.

Fluoride containing compounds are also conventionally incorporated in liquid dentifrices as anticaries agents, and it has been established that these compounds are effective to reduce the incidence of dental caries. Liquid dentifrices sold over the counter conventionally contain fluoride compounds capable of releasing about 500 to 1100 parts per million (ppm) fluoride ion.

Patients with high risk dental caries are frequently prescribed prescription anticaries dentifrice paste products which contain fluoride salts capable of releasing 3 to 5 times the amount of fluoride ion as conventionally incorporated in over the counter anticaries products.

Fluoride compounds which are deemed to be the most effective as anticaries agents are fluoride salts such as sodium fluoride, sodium monoflurophosphate and stannous fluoride. The fluoride salts are effective mainly due to the release of fluoride ions which improve the acid resistance of tooth enamel and accelerate recalcification of decayed teeth in their early stage when the decalcification has proceeded only slightly. The effect of improving the acid resistance of the enamel is believed to be due to the fact that the fluoride ions released by fluoride compounds are incorporated into a crystal lattice of hydroxyapatite which is the main constituent of tooth enamel or, in other words, fluoride ions partially fluoridate hydroxyapatite and simultaneously repair the lattice irregularities.

The effectiveness of fluoride treatment in providing enamel acid resistance is dependent upon the amount of fluoride ion which is available for deposition on the enamel being treated. It is, therefore, desirable to formulate dentifrice compositions which provide optimum fluoride ion availability to tooth enamel using the dentifrice products containing high levels of fluoride compounds.

### SUMMARY OF THE INVENTION

In accordance with the present invention there is provided a method for optimizing fluoride ion uptake on teeth which comprises preparing a liquid dentifrice composition comprised of an orally acceptable vehicle which contains an abrasive and a fluoride ion releasable fluoride compound, the fluoride compound being present in the vehicle at a concentration of the compound sufficient to release 3000 to 6000 ppm fluoride ion wherein the viscosity of the composition ranges from about 10,000 to about 50,000 centipoise per second (cps).

It has been unexpectedly determined that by administering the composition as by brushing on the teeth, a liquid dentifrice composition prepared at the aforementioned viscosity range such compositions are effective to optimize the uptake of fluoride ion on the teeth so as to enhance the anticaries efficacy of the administered fluoride compound.

### DESCRIPTION OF THE PREFERRED EMBODIMENTS

The vehicle of the liquid dentifrice of the present invention is comprised of about 75 to about 90% by weight of a mixture of water and humectants, the liquid composition being adjusted with abrasive thickening agents blended therein to a viscosity of about 10,000 to about 50,000 cps.

The liquid dentifrice of the present invention is easily applicable to toothbrush bristles and when applied thereto does not quickly soak through the bristles, but remains on the top of bristles until the user inserts the brush into the mouth for brushing. A desired quantity of liquid dentifrice can be dispensed from a plastic bottle or other flexible container simply by squeezing the container walls to dispense the liquid dentifrice product. In addition, the liquid dentifrice of the present invention is readily dispersible in the mouth and can be easily washed away from toothbrush bristles.

In general the liquid vehicle for the dentifrice comprises water, in an amount ranging from about 40 to about 60% by weight and preferably about 35 to 45% by weight and a humectant such as glycerin, sorbitol, polyethylene glycol or a mixture thereof in an amount ranging from 25 to 45% by weight and preferably about 30 to about 40% by weight.

Fluoride ion releasable compounds suitable for use in the practice of the present invention include alkali metal fluoride salts such as sodium fluoride, sodium monofluorophosphate and tin salts such as stannous fluoride. The fluoride salt is incorporated in the dentifrice composition at a concentration of about 0.75 to about 2.0% by weight, and preferably at about 0.90 to about 1.3% by weight. At these preferred concentrations about 3000 ppm to about 6000 ppm fluoride ion and most preferably about 4000 to about 5500 ppm fluoride ion will be available to teeth when the liquid dentifrice composition brushed on the teeth.

Surfactants are used in the liquid dentifrice compositions of the present invention to achieve increased prophylactic action and render the compositions more cosmetically acceptable. The surfactant is preferably a detersive material which imparts to the compositions detersive and foaming properties. Suitable examples of surfactants are water soluble salts of higher fatty acid monoglyceride monosulfates, such as the sodium salt of the monosulfated monoglyceride of hydrogenated coconut oil fatty acids, hiher alkyl sulfates such as sodium lauryl sulfate, alkyl aryl sulfonates such as sodium dodecyl benzene sulfonate, higher alkyl sulfoacetates, sodium lauryl sulfoacetate and higher fatty acid esters of 1,2-dihydroxy propane sulfonate. The surfactant is typically present in the dentifrice compositions of the present invention in an amount of about 0.75 to about 3% by weight, preferably about 1.0 to about 2% by weight.

Thickening agents commonly used in dentifrice compositions such as xanthan, carboxymethyl cellulose and polyoxyethylene polyoxypropylene glycol block copolymers and inorganic silica thickeners such as amorphous precipitated silica marketed by J.H. Huber Company under the trade designation Zeodent 165, are used at a concentration of about 0.5 to about 2% in the preparation of the dentifrice composition of the present invention.

An abrasive is generally included in the dentifrice composition of the present invention at a concentration of about 3 to about 25% by weight and preferably at a concentration of about 5 to about 20% by weight. Suitable abrasives include precipitated amorphous hydrated silica, such as Sorbosil AC- 35 available from Crosfield Chemicals, Zeodent 115 or Silodent XWA available from J .M. Huber Company, sodium metaphosphate, potassium metaphosphate, tricalcium phosphate, calcium phosphate dihydrate, anhydrous dicalcium phosphate, calcium pyrophosphate, sodium bicarbonate, calcium carbonate and calcined alumina.

Pyrophosphate salts may be included in the liquid dentifrice compositions as antitartar agents. Exemplary antitartar pyrophosphate salts include dialkali or tetraalkali metal pyrophosphate salts such as tetrasodium pyrophosphate, tetrapotassium pyrophosphate and mixtures thereof, long chain polyphosphates such as sodium hexametaphosphate, sodium tripolyphosphate and cyclic phosphates such as sodium trimetaphosphate. These antitartar salts are included in the dentifrice composition of the present invention at a concentration of about 1 to about 7% by weight.

Synthetic anionic polycarboxylates may also be used in the dentifrice compositions of the present invention to enhance the efficacy of specific active agents present in the dentifrice such as antitartar pyrophosphate salts. These anionic polycarboxylates are generally employed in the form of their free acids or preferably partially or more preferably fully neutralized water soluble alkali metal, e.g., potassium and preferably sodium or ammonium salts. Preferred polycarboxylate compounds are 1:4 to 4: 1 copolymers of maleic anhydride or acid with another polymerizable ethylenically unsaturated monomer, preferably methyl vinyl ether/maleic anhydride having a molecular weight (M. W.) of about 30,000 to about 1,000,000 most preferably about 30,000 to about 500,000. These copolymers are commercially available, for example, under the trade designation, as Gantrez, e.g. AN 139 (M.W. 500,000), AN 119 (M.W. 250,000); and preferably S-97 Pharmaceutical Grade (M.W. 70,000). The synthetic anionic carboxylates may be included in the liquid dentifrice composition of the present invention at a concentration of about 0.5 to about 5% by weight.

Other ingredients which may be incorporated in the components of the present invention include pigments, dyes, flavoring and sweetening materials.

Pigments used in the practice of the present invention include non-toxic, water insoluble inorganic pigments such as titanium dioxide, titanium dioxide coated mica and chromium oxide greens, ultramarine blues and pinks and ferric oxides as well as water insoluble dye lakes. The pigments have a particle size in the range of 5-1000 microns, preferably 250-500 microns, and are present at a concentration of 0.5 to 3% by weight.

The dyes used in the practice of the present invention are distributed uniformly throughout the dentifrice component and are generally food color additives presently certified under the Food Drug & Cosmetic Act for use in food and ingested drugs, including dyes such as FD&C Red #3 (sodium salt of tetraiodofluorescein), FD&C Yellow #5 (sodium salt of 4-p-sulfophenylaxo-B-naphtol-6-monosulfonate), FD&C Green #3 (disodium salt of 4-{[4-(N-ethyl-p-sulfobenzylamino)-phenyl]-4-hydroxy-2-sulfoniumphenyl)-methylene}-[1-(N-ethyl-N-p-sulfobenzyl)-3,5-cyclohexadienimine], FD&C Blue #1 (disodium salt of dibenzyldiethyl-diaminotriphenylcarbinol trisulfonic acid anhydrite), FD&C Blue #2 (sodium salt of disulfonic acid of indigotin) and mixtures thereof in various proportions. The concentration of the dye for the most effective result in the present invention is present in an amount from about 0.0005% to about 2% by weight.

Any suitable flavoring or sweetening agents may also be incorporated in the liquid dentifrice composition of the present invention. Examples of suitable flavoring agents are flavoring oils, e.g., oils of spearmint, peppermint, wintergreen, sassafras, clove, sage, eucalyptus, marjoram, cinnamon, lemon, orange, and methyl salicylate. Suitable sweetening agents include sucrose, lactose, maltose, sorbitol, xylitol, sodium cyclamate, perillartine, and sodium saccharin. Suitably, flavor and sweetening agents may together comprise from 0.01% to 5% by weight or more of the preparations.

Additional other materials may be incorporated in the liquid dentifrice composition of this invention, including buffering agents such as sodium phosphate, stabilizers such as sodium benzoate, antibacterial agents such as triclosan, chlorhexidene, desensitizers such as potassium nitrate and potassium citrate, whitening agents such as hydrogen peroxide, calcium peroxide and urea peroxide, silicones, and chlorophyll compounds.

To prepare the liquid dentifrice composition of the present invention, water, humectant, e.g., sorbitol, thickener and sweetener are dispersed in a conventional mixer until the mixture becomes a homogeneous gel phase. Into the gel phase are added a fluoride anticaries agent such as sodium fluoride. These ingredients are mixed until a homogeneous phase is obtained. Thereafter the abrasive, flavor and surfactant ingredients are added and the ingredients mixed at high speed under vacuum of about 20-100 mm Hg to prepare the final liquid dentifrice product.

The following Example illustrates the present invention. The liquid dentifrice compositions described below were prepared by following the procedures described above. The amounts of the various ingredients are by weight unless otherwise indicated.

### Example

A liquid dentifrice composition of the present invention, designated "Dentifrice A" was prepared containing the ingredients listed in Table I below. Dentifrice A had a viscosity of 11,183 cps. Viscosity measurements in centipoise per seconds (cps) were determined using a Brookfield Viscometer Model LVT. Measurements were taken at 6 revolutions per minute (rpm) with spindle E at room temperature (23°C). Viscosity readings were recorded for a total of 2.5 minutes. The readings were calculated into "cps" from Brookfield units and reported as the viscosity of the liquid dentifrice.

For purposes of contrast, the procedure of the Example was repeated with the exception that a paste dentifrice designated Dentifrice B containing the same fluoride ion releasable salt (NaF) as Dentifrice A at the same weight concentration (1.105% by weight) having a viscosity of 90,000 cps was also prepared. The ingredients of comparative Dentifrice B are also listed in Table I below. Both Dentifrice A and C were evaluated for tooth enamel fluoride uptake and fluoride ion release.

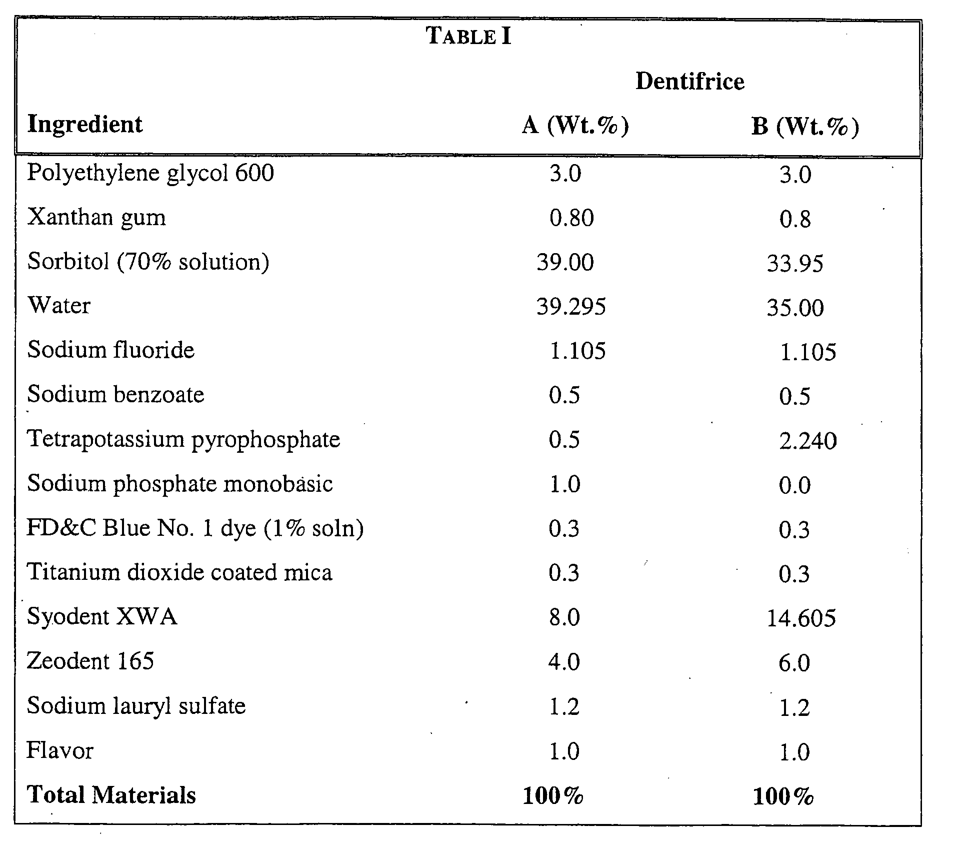

### Fluoride Uptake

Fluoride uptake values were determined by FDA Method #40 wherein one part dentifrice and 3 part water slurry are mixed, centrifuged and the supernatant exposed to an enamel specimen. After 30 minutes one layer of enamel was removed from the specimen with acid and measured for fluoride content.

Fluoride uptake value from the liquid Dentifrice A was 1087 ppm F whereas fluoride uptake from the paste Dentifrice B was substantially less namely, 754 ppm F.

### Fluoride Release

Fluorine ion release was measured wherein a 5 gram amount of Dentifrice A and B were dispersed into 15 grams of water using a test procedure similar to that of the ADA 1:3 Fluoride Availability Test requirement wherein the amount of fluoride ion present in solution was measured every 5 seconds for one minute for a total of 12 readings using a fluoride ion specific reference electrode. The results of the fluoride release studies of Dentifrices A and B are recorded in Table II below.

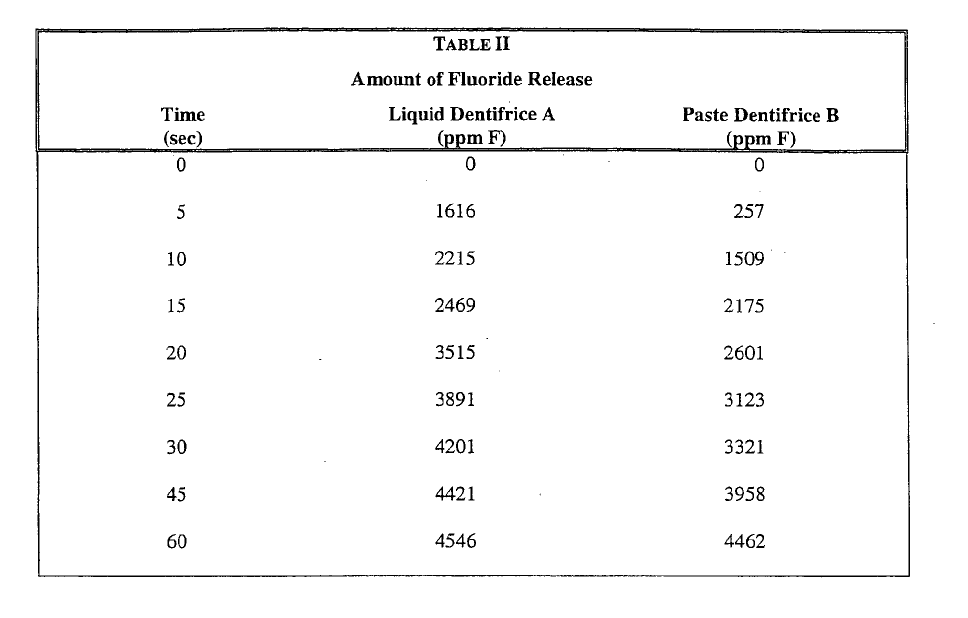

Reference to Table II indicates that the liquid formula Dentifrice A as compared to paste Dentifrice C exhibited continuously greater fluoride release over the 60 second measurement period demonstrating enhanced fluoride ion bioavilablility available from the liquid dentifrice composition during a one minute period normally employed during a tooth brushing regimen.

## Claims

1. A method for optimizing fluoride ion uptake on teeth which comprises preparing a liquid dentifrice composition comprised of an orally acceptable vehicle containing an abrasive and a fluoride ion releasable fluoride salt, the fluoride salt being present in the vehicle at a concentration sufficient to release 1000 to 6000 ppm fluoride ion, adjusting the viscosity of the composition to a range of about 10,000 to about 50,000 cps and then administering the composition by brushing the composition on the teeth, whereby the uptake of fluoride ion on the teeth is optimized so as to enhance the anticaries efficacy of the administered dentifrice composition.

2. The method of claim 1 wherein the liquid dentifrice composition has a viscosity in the range of about 10,000 to about 50,000 cps.

3. The method of claim 1 wherein the fluoride salt is sodium fluoride.

4. The method of claim 1 wherein the abrasive is silica.

5. The method of claim 1 wherein the amount of fluoride ion releasable from the fluoride salt is in the range of about 4000 to 5500 ppm.
